# DEMANDE DE BREVET EUROPEEN

(11) **EP 1 201 748 A2**
(43) Date de publication de la demande: **02.05.2002**
(21) Numéro de dépôt: 01402680.1
(22) Date de dépôt: 17.10.2001
(51) Int. Cl.: C12N 1/20, C12N 1/38, C12N 1/04, A23C 9/12

(54) **Procédé de production de ferments alimentaires**

(30) Priorité: 30.10.2000 FR 0013956
(71) Demandeur: Roquette Frères, 62136 Lestrem (FR)
(72) Inventeur: Ostermann, Elsa, 62400 Bethune (FR); Pora, Bernard, 59151 Arleux (FR); Boursier, Bernard, 62138 Violaines (FR); Defretin, Sophie, 62400 Locon (FR); Wils, Daniel, 59190 Morbecque (FR)
(74) Mandataire: Boulinguiez, Didier

(57) **Abrégé**

L'invention concerne un procédé de préparation de ferments alimentaires comprenant une fermentation de bactéries lactiques dans un milieu de culture comprenant au moins un saccharide activateur de fermentation choisi dans le groupe constitué par les maltodextrines, les maltodextrines branchées, les amidons solubles, les oligosaccharides, seuls ou en mélange.

Elle a également pour objet un procédé de conservation de ferments alimentaires caractérisé en ce qu'il met en oeuvre au moins un saccharide activateur de fermentation choisi dans le groupe constitué par les maltodextrines, les maltodextrines branchées, les amidons solubles, les oligosaccharides, seuls ou en mélange.

## Description

La présente invention a pour objet un procédé de production de ferments alimentaires par fermentation de bactéries lactiques dans un milieu de culture contenant un saccharide particulier en tant qu'activateur de fermentation. Elle a également pour objet un procédé de conservation de ferments alimentaires mettant en oeuvre ledit saccharide particulier. Les ferments alimentaires visés par la présente invention sont des bactéries à Gram positif, parmi lesquelles on retrouve les bactéries lactiques qui se caractérisent par une forte production d'acide lactique, et les bactéries bifides qui sont apparentées aux bactéries lactiques et qui se caractérisent par une fermentation mixte, lactique et acétique.

Les bactéries lactiques comprennent les genres suivants : *Lactobacillus, Lackococcus, Streptococcus, Enterococcus, Leuconostoc, Pediococcus.* Les bactéries bifides sont représentées par le genre *Bifidobacterium.*

On trouve ces bactéries généralement associées à d'autres microorganismes dans de nombreux produits de fermentation naturelle animaux et végétaux : laits fermentés (fromages, yaourts), viandes fermentées (saucisson, jambon), boissons alcoolisées à base de fruits (vins, cidres, bières), légumes et fruits fermentés (choucroute, olives, cornichons), céréales fermentées (différentes variétés de pains), fourrages fermentés (ensilage).

Ces bactéries, que l'on regroupera sous le vocable « ferments alimentaires » sont considérées comme un des groupes bactériens les plus exigeants du point de vue nutritionnel (La technique laitière N°979, 1983 - pp 41-47).

Les bactéries lactiques sont depuis longtemps largement employées dans la conservation des aliments à base de lait, de viande, de poisson, de légumes et de fruits. L'innovation la plus marquante en matière de préparation de ferments aura été l'introduction de ferments concentrés sous forme congelée, et peu après, lyophilisée. Ces ferments concentrés simplifient beaucoup la tâche des utilisateurs et favorisent l'obtention d'une fabrication plus régulière.

Les caractéristiques du milieu de culture destiné à la production industrielle de ferments lactiques conditionnent très largement l'activité finale du produit. Malgré les exigences nutritionnelles des bactéries lactiques, les milieux de culture doivent permettre d'obtenir une biomasse concentrée avec de bons rendements de production. Les besoins en nutriments sont satisfaits en fournissant aux microorganismes une ou plusieurs sources azotées, une source carbonée, des vitamines, des minéraux, et dans certains cas des acides gras, des bases azotées et des acides organiques. Généralement, il est recommandé d'utiliser les mêmes substrats carbonés que ceux contenus dans les milieux auxquels les ferments sont destinés. Il est souvent préférable d'utiliser des disaccharides tels que le lactose, le saccharose ou le maltose pour obtenir une bonne activité finale du ferment.

C'est en s'intéressant à cette question que la Demanderesse a constaté que l'utilisation d'une famille de saccharides particuliers dans un procédé de production de ferments alimentaires avait, de façon surprenante et inattendue, un effet stimulant sur la croissance des ferments et par conséquent sur la vitesse d'acidification du milieu de croissance. Ceci est particulièrement étonnant puisqu'il est connu que les sources de carbone de faible poids moléculaire comme notamment le lactose sont préférentiellement métabolisées par les bactéries lactiques.

L'invention a donc pour objet un procédé de production de ferments alimentaires comprenant une fermentation de bactéries lactiques dans un milieu de culture comprenant au moins un saccharide activateur de fermentation choisi dans le groupe constitué par les maltodextrines, les maltodextrines branchées, les amidons solubles, les oligosaccharides, seuls ou en mélange. Selon une variante préférée du procédé selon l'invention, ledit activateur de fermentation est choisi dans le groupe constitué par les maltodextrines branchées, les amidons solubles, les oligosaccharides, seuls ou en mélange. Selon une autre variante préférée du procédé selon l'invention, ledit activateur de fermentation est choisi dans le groupe constitué par les amidons solubles et les maltodextrines branchées.

Ce procédé de fermentation peut aussi bien être appliqué in vitro que in vivo. Le saccharide activateur de fermentation conforme à l'invention peut ainsi favoriser la croissance in vivo des flores lactiques humaines ou animales.

Par « maltodextrines » on entend les maltodextrines standard obtenues classiquement par hydrolyse acide et/ou enzymatique de l'amidon, et caractérisées par un pouvoir réducteur exprimé en Dextrose Equivalent (ou D.E) inférieur à 20.

Par « maltodextrines branchées » on entend les maltodextrines décrites dans la demande de brevet FR-A-2.786.775 dont la Demanderesse est titulaire. Ces maltodextrines sont caractérisées par une teneur en liaisons glucosidiques 1→6 supérieure à celle des maltodextrines standards. Elles présentent entre 15 et 35% de liaisons glucosidiques 1→6, une teneur en sucres réducteurs inférieure à 20%, un indice de polymolécularité inférieur à 5 et une masse moléculaire moyenne en nombre Mn au plus égale à 4500 g/mole. Elles peuvent également être hydrogénées.

Par « amidons solubles » on entend les amidons de toute origine modifiés physiquement ou chimiquement et susceptibles d'être solubles dans l'eau à température ambiante.

Par oligosaccharides on entend notamment les fructooligosaccharides, les isomaltooligosaccharides, les maltooligosaccharides, les galactooligosaccharides, les xylooligosaccharides, les palatinose oligosides, le lactulose, le raffinose et le lactosucrose.

De préférence, pour obtenir l'effet stimulant recherché dans la préparation des ferments, le procédé selon l'invention est caractérisé en ce que le saccharide activateur est présent dans le milieu de culture à une concentration au moins égale à 0,1%.

Selon une variante préférée du procédé selon l'invention, la fermentation est effectuée avec des bactéries lactiques choisies parmi les genres *Lactobacillus delbrueckii sp. Bulgaricus, Streptococcus thermophilus*.

Avantageusement, le milieu de culture utilisé dans le procédé selon l'invention contient 0,1 à 30%, de préférence 0,1 à 25% et plus préférentiellement encore 0,5 à 15% en poids d'au moins un saccharide activateur de fermentation choisi dans le groupe constitué par les maltodextrines, les maltodextrines branchées, les amidons solubles, les oligosaccharides, seuls ou en mélange.

Le procédé conforme à l'invention pourra avantageusement être appliqué à la transformation de denrées alimentaires par fermentation, et notamment pour la préparation de laits fermentés et autres spécialités laitières, puisque le saccharide activateur, non entièrement métabolisé par les ferments, apportera aux produits finis des propriétés rhéologiques et nutritionnelles avantageuses. La Demanderesse a en effet constaté que le procédé conforme à l'invention appliqué à la préparation de spécialités laitières non seulement augmentait la vitesse de fermentation mais aussi améliorait la texture finale de celles-ci. De plus, lorsqu'on choisit le saccharide activateur parmi les oligosaccharides qui contiennent des fibres solubles, celui-ci peut conférer avantageusement des propriétés bifidogènes aux produits finis et constitue un apport en fibres solubles.

Le procédé selon l'invention peut également être appliqué à la préparation de compositions pour l'alimentation animale, comme l'ensilage notamment.

C'est pourquoi l'invention a également pour objet un procédé de préparation de compositions alimentaires fermentées caractérisé en ce qu'il comprend au moins une étape consistant à mettre en oeuvre un procédé de production de ferments alimentaires conforme à l'invention.

La présente invention a en outre pour objet un procédé de conservation de ferments alimentaires caractérisé en ce qu'il met en oeuvre au moins un saccharide activateur de fermentation choisi dans le groupe constitué par les maltodextrines, les maltodextrines branchées, les amidons solubles, les oligosaccharides seuls ou en mélange. La Demanderesse a en effet constaté que ledit saccharide activateur assurait une viabilité satisfaisante des bactéries lors de la conservation. Le saccharide activateur peut donc avantageusement être utilisé comme support de conservation par séchage, atomisation, congélation, lyophilisation des ferments. Il pourra ainsi, lors de la remise en solution des ferments, jouer à nouveau son rôle d'activateur de fermentation.

D'autres avantages apparaîtront clairement à la lecture des exemples qui suivent, qui se veulent illustratifs et non limitatifs.

### Exemple 1 : application aux laits fermentés.

On prépare un lait fermenté en utilisant les ferments lactiques traditionnels (*Lactobacillus delbrueckii sp. Bulgaricus* et *Streptococcus thermophilus*) et on suit la fermentation par mesure de la variation d'impédance du milieu au cours du temps d'incubation (BACTOMETER®, BIOMERIEUX) et par la détermination quantitative et qualitative de la population microbienne par cytométrie de flux (CHEMFLOW®, CHEMUNEX). Le système BACTOMETER® est un système de détection microbienne rapide, fiable et sensible et entièrement automatisé, reposant sur les principes de la microbiologie par impédance. Il est conçu pour détecter les changements d'impédance résultant de l'activité métabolique des microorganismes en croissance. Le temps de détection mesuré est fonction à la fois de la cinétique de croissance, du temps de latence et de la concentration en microorganismes d'un échantillon donné. Les résultats sont exprimés en TD(temps de détection). L'influence de différents saccharides sur la croissance des ferments lactiques est étudiée.

Le milieu de croissance est d'un volume de lml, composé de 9% de lait, 10% de saccharose et/ou 10% de saccharides à étudier. Le lait est préparé par réhydratation de lait en poudre dans de l'eau stérile, il n'est pas pasteurisé. Les saccharides à étudier sont stérilisés par filtration.

La suspension de ferments lactiques est préparée dans de l'eau stérile afin d'obtenir une population initiale de 2 10⁶ UFC/ml de milieu lacté.

L'incubation se fait à 44°C pendant 24 heures. La variation d'impédance du milieu est mesurée en continu et est traduite en % de variation.

Les résultats sont consignés dans le tableau ci-après.

| **saccharide** | **Concentration (%)** | **Temps de détection en heures** |
|---|---|---|
| Aucun | | 4,6 |
| Saccharose | 10 | 4,6 |
| Glucose | 10 | 14,8 |
| GLUCIDEX®47 | 10 | 4,2 |
| GLUCIDEX®21 | 10 | 3,8 |
| GLUCIDEX®6 | 10 | 3,3 |
| Amidon soluble* | 10 | 3,4 |
| Galactooligosaccharides** | 10 | 4,1 |
| Isomaltooligosaccharides*** | 10 | 4,1 |
| Fructooligosaccharides**** | 10 | 3,8 |
| Maltodextrines branchées | 10 | 4,1 |

| | | |
|---|---|---|
| Les GLUCIDEX® sont des maltodextrines standards commercialisées par la Demanderesse. *L'amidon soluble est un amidon hydrolysé par voie acide, commercialisé par la société MERCK sous la référence 1.01252. | | |
| **Galactooligosaccharides : Cup Oligo P® - Nisshin Seito | | |
| ***Isomaltooligosaccharides : Isomalt®900P - Showa Sangyo | | |
| ****Fructooligosaccharides : Actilight®950P - Beghin-Meiji Maltodextrines branchées : on utilise une maltodextrine présentant entre 15 et 35% de liaisons glucosidiques 1→6, une teneur en sucres réducteurs comprise entre 2 et 5% et un Mn compris entre 2000 et 3000 g/mole. | | |

Les résultats montrent que l'effet positif des saccharides activateurs selon l'invention n'est pas relatif à une diminution de la pression osmotique puisque le temps de détection est identique au témoin en l'absence de saccharide additionnel au lactose du lait.

Une diminution du temps de détection correspondant à une diminution du temps de latence est observée pour les essais où le saccharose a été substitué en particulier par les maltodextrines (GLUCIDEX®), l'amidon soluble, les maltodextrines branchées, les fructooligosaccharides.

### Exemple 2 : détermination de la concentration bactérienne.

Les ferments lactiques ont été mis en oeuvre sur les milieux décrits dans l'exemple précédent (lait 9% et saccharose 10%, ou saccharide activateur 10%).

L'incubation a été faite à 44°C en réacteur de 2 litres avec suivi cinétique de l'acidification. Des prélèvements ont été effectués au cours de la fermentation afin de déterminer la concentration bactérienne totale et la viabilité de cette population grâce à l'analyseur CHEMFLOW®.

Les fermentations ont été stoppées dès que le pH a atteint une valeur de 4,4.

Le tableau suivant indique les concentrations en cellules totales et les concentrations en cellules vivantes, le rapport des deux valeurs donnant la viabilité cellulaire. Les analyses des derniers prélèvements correspondant aux fins de fermentations, ont été effectués après brassage des yaourts. Les valeurs sont exprimées en cellules comptées par ml de milieu fermenté.

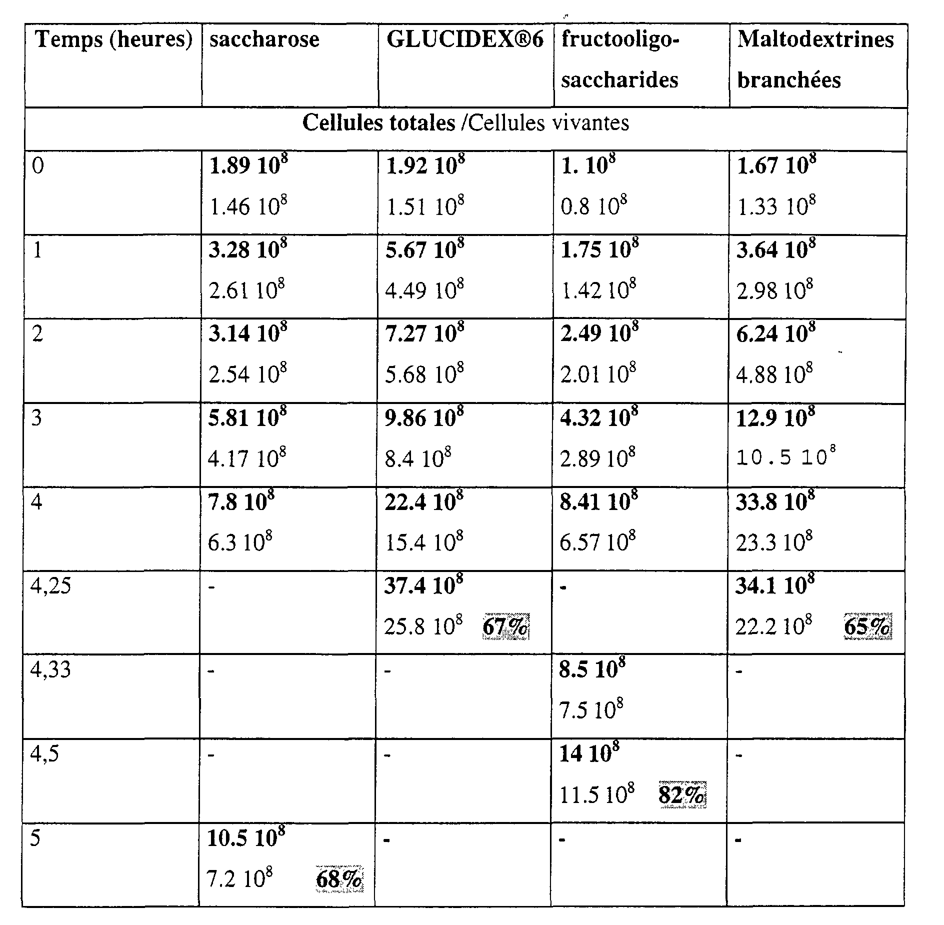

Les viabilités pour chacun des milieux sont calculées en effectuant le rapport entre cellules vivantes et cellules totales, exprimé en pourcentage. Les viabilités sont respectivement de 68, 67, 82 et 65%. Selon le saccharide activateur utilisé, les concentrations cellulaires viables sont 1.6 à 3.6 fois plus concentrées que le témoin saccharose, les GLUCIDEX®6 et les maltodextrines branchées étant les plus activateurs de croissance. En effet, le temps nécessaire pour obtenir un pH de 4,4 est nettement plus court : 4,25 heures avec GLUCIDEX®6 et maltodextrines branchées, 4,5 heures avec les fructooligosaccharides, alors qu'il faut 5 heures lorsqu'on utilise le saccharose.

### Exemple 3

Des essais ont été réalisés dans les conditions de l'exemple 1 mais avec variation des concentrations en GLUCIDEX®, sans saccharose et avec ajout d'édulcorants intenses.

Les résultats sont repris dans le tableau ci-dessous :

| **SACCHARIDE** | **CONCENTRATION (%)** | **Temps de détection (heures)** |
|---|---|---|
| Saccharose | 10 | 3.9 |
| GLUCIDEX®6 | 0 | 3.9 |
| | 2 | 3.4 |
| | 4 | 3.1 |
| | 6 | 2.9 |
| | 8 | 2.7 |
| | 10 | 2.7 |
| GLUCIDEX®6 + édulcorants | 10 | 2.7 |

Une diminution de 30% du temps de latence est observée pour des concentrations en GLUCIDEX®6 supérieures à 8%. L'ajout d'édulcorants intenses ne modifie pas ce résultat.

Lorsque l'on fait varier les concentrations en GLUCIDEX®6, et en saccharose pour une concentration totale de 10 %, on obtient les résultats suivants :

| **SACCHAROSE (%)** | **GLUCIDEX®6 (%)** | **Temps de détection (heures)** |
|---|---|---|
| 10 | 0 | 3.9 |
| 8 | 2 | 3.7 |
| 6 | 4 | 3.3 |
| 4 | 6 | 2.9 |
| 2 | 8 | 2.6 |
| 0 | 10 | 2.4 |

L'utilisation de saccharides activateurs selon l'invention, dans le milieu de fermentation permet :
- une diminution du temps de latence de la croissance bactérienne,
- une acidification plus rapide avec un gain de temps de fermentation nécessaire à l'obtention d'une valeur de pH de 4,4,
- une population bactérienne 2 à 4 fois plus importante en nombre et présentant une viabilité élevée.

## Revendications

1. Procédé de production de ferments alimentaires comprenant une fermentation de bactéries lactiques dans un milieu de culture comprenant au moins un saccharide activateur de fermentation choisi dans le groupe constitué par les maltodextrines, les maltodextrines branchées, les amidons solubles, les oligosaccharides, seuls ou en mélange.

2. Procédé selon la revendication 1 **caractérisé en ce que** ledit saccharide activateur est présent dans le milieu de culture à une concentration au moins égale à 0,1% en poids.

3. Procédé selon l'une ou l'autre des revendications 1 et 2 **caractérisé en ce que** les bactéries lactiques sont choisies parmi les genres *Lactobacillus delbrueckii sp. Bulgaricus* et *Streptococcus thermophilus.*

4. Procédé selon l'une quelconque des revendications 1 à 3, **caractérisé en ce que** le milieu de culture contient 0,1 à 30%, de préférence 0,1 à 25% et plus préférentiellement encore 0,5 à 15% en poids d'au moins un saccharide activateur de fermentation choisi dans le groupe constitué par les maltodextrines, les maltodextrines branchées, les amidons solubles, les oligosaccharides, seuls ou en mélange.

5. Procédé de préparation de compositions alimentaires fermentées **caractérisé en ce qu'**il comprend au moins une étape consistant à mettre en oeuvre un procédé selon l'une quelconque des revendications 1 à 4.

6. Composition alimentaire susceptible d'être obtenue conformément au procédé selon la revendication 5.

7. Composition alimentaire selon la revendication 6 **caractérisée en ce qu'**elle est un lait fermenté.

8. Procédé de conservation de ferments alimentaires **caractérisé en ce qu'**il met en oeuvre au moins un saccharide activateur de fermentation choisi dans le groupe constitué par les maltodextrines, les maltodextrines branchées, les amidons solubles, les oligosaccharides, seuls ou en mélange.

9. Procédé de conservation selon la revendication 8, **caractérisé en ce qu'**il est une lyophilisation.

10. Procédé de production de ferments alimentaires comprenant une fermentation de bactéries lactiques dans un milieu de culture comprenant au moins un saccharide activateur de fermentation choisi dans le groupe constitué par les maltodextrines branchées, les amidons solubles, les oligosaccharides, seuls ou en mélange.
